# EUROPEAN PATENT APPLICATION

(11) **EP 1 757 938 A1**
(43) Date of publication of application: **28.02.2007**
(21) Application number: 05741432.8
(22) Date of filing: 19.05.2005
(51) Int. Cl.: G01N 33/573, G01N 33/493

(54) **METHOD OF EXAMINING INTERSTITIAL CYSTITIS**

(30) Priority: 20.05.2004 JP 2004149925; 30.03.2005 JP 2005099382
(71) Applicant: Astellas Pharma Inc., Tokyo 103-8411 (JP); Yamada, Tetsuo, Sagamihara-shi, Kanagawa 229-0011 (JP); Mita, Haruhisa, Tokyo 144-0035 (JP)
(72) Inventor: YAMADA, Tetsuo, Sagamihara-shi Kanagawa 229-0011 (JP); MITA, Haruhisa, Ota-ku, Tokyo 144-0035 (JP); KUROMITSU, Sadao c/o ASTELLAS PHARMA Inc., Tokyo 103-8411 (JP); YOKOTA, Hiroyuki c/o ASTELLAS PHARMA Inc., Tokyo 103-8411 (JP); MORITA, Shuji c/o ASTELLAS PHARMA Inc., Tokyo 103-8411 (JP); HIRAMOTO, Masashi c/o ASTELLAS PHARMA Inc., Tokyo 103-8411 (JP); YURI, Masatoshi c/o ASTELLAS PHARMA Inc., Tokyo 103-8411 (JP)
(74) Representative: Bates, Philip Ian
(86) International application number: PCT/JP2005/009153
(87) International publication number: WO 2005/114196

(57) **Abstract**

It has been found that the urine from an IC patient shows a high value in amount and the existence of activity of an azurophilic granular substance, thereby to establish a method for examining IC. The present invention relates to a method for examining interstitial cystitis using the kinetics of an azurophilic granular substance in urine as a marker. Also, the present invention relates to a kit for examining interstitial cystitis for use in the examination method, a use of an azurophilic granular substance as a test marker for examining interstitial cystitis or evaluating pharmacological effects of a drug, and a method for examining therapeutic effects on a patient with interstitial cystitis using an azurophilic granular substance as a marker.

## Description

### Technical Field

The present invention relates to a method for examining interstitial cystitis. More specifically, the present invention relates to a method for examining interstitial cystitis using the kinetics of an azurophilic granular substance of a subject as a marker. The present invention also relates to a kit for examining interstitial cystitis for use in the examination method, a use of an azurophilic granular substance as a marker for examining interstitial cystitis or evaluating the pharmacological effect of a drug, and a method for examining therapeutic effects on a patient with interstitial cystitis using an azurophilic granular substance as a marker.

### Background Art

Interstitial cystitis (hereinafter abbreviated as IC) is one of bacteria-free chronic cystitis that principally characterized by strong bladder pain on filling of bladder and frequent urination, and is an intractable disease which is pathologically unclear and for which no effective therapy is found. Though it is not a fatal disease, it torments a patient with severe pain and frequent urination to damage significantly the patient's QOL. Thus, there is earnestly desired a therapy, a prophylaxis, or a therapeutic agent to develop against the disease. In the examination of the disease, in order to exclude a possible infection case from the disease, the urine from a patient is cultured and judged on whether it is infected with any bacteria. Further, after the patient is confirmed to be free from any other disease (such as bladder cancer, renal disease, tuberculosis, vaginal infection, venereal disease, endometriosis, radiation cystitis and neurosis), cystoscopy with hydrodistension is conducted under general anesthesia. This examination is conducted by observing glomerulation on the bladder wall.
As stated above, the examination of IC, in the present situation, requires a pile of complicated examinations and cystoscopy which is accessible only to a specialist. Under such circumstances, concerning many test markers, reports have been made to analyze and review (non-patent document 1). However, there has not yet been found out an examination method which is acknowledged to be IC-selective and reliable. To find out a marker is very important, because the marker would be useful for the examination as well as allow evaluation of the pharmacological effects of a drug in clinical trials and selection of the adequate therapy depending on a disease condition.
By a recent report relating to IC test markers, it was demonstrated from a blind comparison study on 13 urinary markers at multiple facilities that APF (antiproliferative factor), HB-EGF (heparin-binding epidermal growth factor-like growth factor), EGF (epidermal growth factor), IGFBP3 (insulin-like growth factor binding protein 3), IL-6 (Interleukin 6), cGMP (cyclic guanosine monophosphate) and methylhistamine were determined with their respective significant differences between IC patient group and symptomless control group (non-patent document 2). This report stated that only the APF activity exhibited almost no duplication of data between both groups. Thus, from reports to date, APF is believed to be the most promising marker which can clearly distinguish IC patients from unaffected patients in examination of IC. However, APF activity has to be detected with the growth inhibitory activity of human bladder epithelial cells (non-patent document 3). Therefore, generalization of APF to use as a test marker is accompanied by many problems, for example, difficulty of comparison among facilities to analyze, because the AFP amount can not be determined by any indicator but the activity, which further cannot help being judge in a complicated way and requires the use of living cells to detect.
Further, though NIDDK (National Institute of Diabetes and Digestive and Kidney Diseases) defines standards for IC researches (non-patent document 4), these standards have been prepared without the intention to clinical examination of patients, and thus include so many exclusions that they are difficult to apply to clinical test (non-patent document 5).
Azurophilic granular substances include, besides myeloperoxidase and elastase, many other enzymes and proteins such as cathepsin G (non-patent document 12), proteinase 3 (non-patent document 13), defensin (non-patent document 14) and bactericidal permeability-increasing protein (non-patent document 15) (non-patent document 16). It is known that they are contained in neutrophil azurophilic granules.
Neutrophil elastase is a strong and protease having low substrate specificity. The destructive activity that the elastase has to tissue is demonstrated by acute alveolar hemorrhage and destruction of interalveolar septum caused by neutrophil elastase that is transbronchially injected into an animal model (non-patent document 6). Further, there have been demonstrated the neutrophil elastase activity increased in alveolus wash solution from patients with ARDS (acute respiratory distress syndrome) as well as a high correlation between the level of neutrophil elastase in plasma and lung hypofunction (non-patent document 7), indicating that neutrophil elastase may induce a pathology characteristic of an acute lung injury such as pulmonary interstitial and alveolar edemas. It is also suggested that a neutrophil elastase inhibitor may serve as a therapeutic agent against ARDS, COPD (chronic obstructive pulmonary disease), and CF (cystic fibrosis), in which neutrophil is suggested to involve itself pathologically (non-patent document 8). Further, the reported examples of body fluid with increased neutrophil elastase include, in addition to the fluid from a patient with acute lung inflammation, the semen of a patient with chronic uninfected prostatitis (non-patent document 9), the plasma of a patient with acute pyelonephritis (non-patent document 10), and the urine of an infected urethritis patient (non-patent document 11).
Myeloperoxidase is a principal protein of azurophilic granule of neutrophil. It catalyzes a reaction of hydrogen peroxide with a chlorine ion to produce hypochlorite for attacking foreign antigens. Meanwhile, the measured myeloperoxidase activity, which can be used as a marker to indicate the activation of a neutrophil, is reported to use for a prognostic prediction factor of myocardial infarction (non-patent document 17), and is reported to be applicable as a marker for glomerulonephritis (non-patent document 18).
On the other hand, concerning IC, there is no report describing that any azurophilic granular substance, in particular neutrophil elastase or myeloperoxidase is found or increased in amount in a bladder tissue or an urine.

[non-patent document 1] Urology 2001, 57: 15-21
[non-patent document 2] J. Urol., 2002, 167: 2461-2469
[non-patent document 3] J. Urol., 1996, 156: 2073-2078
[non-patent document 4] J. Urol., 1988, 140: 203-206
[non-patent document 5] J. Urol., 1999, 161: 553-557
[non-patent document 6] Am. J. Pathol., 1979, 97: 111-136
[non-patent document 7] Am. Rev. Respir. Dis. 1985, 132: 1098-1105
[non-patent document 8] Idrugs., 2002, 5: 910-923
[non-patent document 9] World J. Urol., 2003, 21: 82-85
[non-patent document 10] Pediatrics, 2000, 105: E28
[non-patent document 11] Genitourin Med., 1995, 71: 176-179
[non-patent document 12] Enzyme, 1972, 12: 132
[non-patent document 13] Am. J. Pathol., 1990, 137: 1113
[non-patent document 14] Blood, 1987, 70: 757
[non-patent document 15] Blood, 1987, 69: 652
[non-patent document 16] Blood 1997, 89: 3503-3521
[non-patent document 17] N. Engl. J. Med., 2003, 349: 1595-1604
[non-patent document 18] Kidney Int., 2003, 64: 1956-1967

### Disclosure of the Invention

### (Problem to be Solved by the Invention)

The subject of the present invention is to provide an easy examination method for IC.

### (Means for Solving the Problem)

The present inventors made a strenuous study to find that neutrophil elastase and myeloperoxidase, which are azurophilic granular substances, are contained in the urine from an IC patient at their high values of amount, and that neutrophil elastase activity is found there. Thus, there has been established an examination method for IC using the amount and/or activity of an azurophilic granular substance (and preferably neutrophil elastase or myeloperoxidase) as markers. In addition, we found a high correlation between pain score and the activity and/or amount of neutrophil elastase which is an azurophilic granular substance, and then have established an examination method for therapeutic effects using the amount and/or activity of an azurophilic granular substance as a marker. Thus, the present invention has been completed.

Therefore, the present invention relates to:
1. A method for examining interstitial cystitis, comprising a step for measuring azurophilic granular substance as a marker.
2. The examination method according to preceding item 1 wherein the azurophilic granular substance is neutrophil elastase.
3. The examination method according to preceding item 1, wherein the azurophilic granular substance is myeloperoxidase.
4. The examination method according to any one of preceding items 1 to 3, wherein the urine sample from a patient with an uninfected urine disease as a subject is used as an analyte for the examination.
5. The examination method according to any one of preceding items 1 to 4, comprising further a step of determining whether the urine sample is infected with a bacteria or not.
6. The examination method according to any one of preceding items 2, 4 and 5, wherein the step of measuring neutrophil elastase as a marker is a step for measuring neutrophil elastase amount.
7. The examination method according to any one of preceding items 2, 4 and 5, wherein the step of measuring neutrophil elastase as a marker is a step for measuring neutrophil elastase activity.
8. The examination method according to preceding item 6, wherein the step for measuring neutrophil elastase amount is a step conducted by immunoassay.
9. The examination method according to preceding item 7, wherein the step for measuring neutrophil elastase activ ty is a step conducted with a synthetic substrate.
10. A kit for examining interstitial cystitis for use in the examination method according to preceding item 8, comprising an assay container and an anti-neutrophil elastase antibody for measuring neutrophil elastase amount.
11. A kit for examining interstitial cystitis for use in the examination method according to preceding item 9, comprising an assay container, a buffer and a neutrophil elastase substrate for measuring neutrophil elastase activity.
12. A use of an azurophilic granular substance as a marker for examining interstitial cystitis or for evaluating pharmacological effects of a drug.
13. A method for examining therapeutic effects on a patient with interstitial cystitis using an azurophilic granular substance as a marker.
   Patent document of US 6566331 is an application relating to a therapeutic method for collagen-associated diseases including diffuse connective tissue disease, lung fibrosis, splenomegaly, interstitial cystitis, scleroderma, angiitis, and describes that it was shown by cystoscopy that α-1-antitrypsin was intravenously infused into the bladder of an interstitial cystitis patient to alleviate the impairment. Further, the patent document of WO 2004/045634, which was published after the priority date of the present application, describes that protease inhibitors, in particular α-1-antitrypsin, may serve as therapeutic agents for dermatitis, lung, ear, eye, and gastrointestinal diseases and uropathy, and that the disease example includes colitis and interstitial cystitis. However, these patent documents never mentioned that azurophilic granular substance (in particular, neutrophil elastase and myeloperoxidase) can work as a test marker for interstitial cystitis, and did not at all disclose a method for examining interstitial cystitis comprising a step of measuring azurophilic granular substance as a marker. As is obviously acknowledged from the description by Essential Urology, 6th ed., Ishiyaku Publishers, Inc. (p.57-61) that an urinary sediment from an interstitial cystitis patient shows no or little change except for erythrocytes observed although an urinary sediment from a bacterial cystit is patient regardless of acute and chronic shows increased leukocytes observed, it has not been known that the amount or activity of neutrophil elastase released from neutrophil, which is a kind of leukocytes, increases in the urine of an interstitial cystitis patient, and also that an urinary sediment from a non-bacterial interstitial cystitis patient shows increase leukocytes.
   Therefore, it is our novel finding that azurophilic granular substance (in particular neutrophil elastase and myeloperoxidase) can work as a test marker for interstitial cystitis, so that a method for examining interstitial cystitis comprising a step of measuring azurophilic granular substance as a marker, a use of an azurophilic granular substance as a test marker for interstitial cystitis or an evaluation marker for pharmacological effects of a drug, and an examination method for therapeutic effects on a patient with interstitial cystitis using an azurophilic granular substance as a marker are inventions provided by the present inventors for the first time.

### (Effects of the Invention)

A marker for a novel examination of IC is established by the present invention. Namely, an azurophilic granular substance in urine of a patient is found to be useful for a marker for IC disease, thereby an examination method for IC using this marker is established, and complexity in conventional IC examination was improved, successfully providing a means allowing a more reliable and easier IC examination.
The usefulness of a diagnostic marker is evaluated by "sensitivity" which can determine a patient as positive, "specificity" which can determine a nonpatient as negative and "diagnostic efficiency" (positive predictive value) which is defined by the product of "sensitivity" and "specificity" (Nippon Rinsho , vol. 54, No. 6: pp. 121-125). Diagnostic efficiency in IC patient obtained by measuring the activity of urinary elastase, which is one of the present invention, was 62% (calculated in Example 4), and Diagnostic efficiency in IC patient obtained by measuring the amount of urinary elastase by enzyme immunoassay was 57% (calculated in Example 5). On the other hand, diagnostic efficiency of a prostate-specific antigen (PSA), which is clinically and broadly used for diagnosis of prostate cancer, determination of therapeutic effects and an indicator of relapse, is calculated from sixteen examples cited in the review (J. Urology, 1998, 159: 5-12) to give a mean value of 36% with a dispersion of 17 to 67%. Therefore, it is found that the examination method for IC of the present invention has a diagnostic efficiency comparable or more to a clinically used examination method and is useful for an examination method.

### Description of the Preferred Embodiment

Terms in the present invention will be explained.
"Bacterial urine" herein means a state in which bacteria are present in urine. Whether bacteria are present in urine or not can be detected by the following method of (1) and/or (2).
(1) An urine sample is collected after wiping and cleaning around an ureteric orifice, diluted appropriately as needed, cultured quantitatively, and measured in respect to its urinary bacterial concentration. The urine, which has a bactericidal concentration of 10⁵ colony forming units (CFUs)/mL or more, is referred to as a bacterial urine.
(2) A function of a certain bacteria (such as E. coli and myxomycetes) to reduce nitrite present in urine is used to determine by a conventional method whether bacteria are present or absent in urine. It is conducted by a test strip method based on grease reaction. A white area gets colored by reddish violet azo dye formed depending on the nitrite concentration of urine, allowing detection of bacterial urine.
   "A patient with uninfected urine disease" herein means a patient with the other urine disease than urinary tract infection. The urinary tract infection means an injected inflammation caused in the urinary tract by a nonspecific bacteria, which is observed in urine.

One of the present invention is an examination method for IC, wherein azurophilic granular substance is used as the marker. The "azurophilic granular substance" herein represents a substance contained in azurophilic granules and is exemplified by, besides myeloperoxidase and elastase, cathepsin G, proteinase 3, Defensin and bactericidal permeability-increasing protein. In urine of an IC patient, there are shown high amounts of those azurophilic granular substances, in particular neutrophil elastase and myeloperoxidase and the activity of neutrophil elastase.
One of specific means in the present invention is an examination method for IC, wherein neutrophil elastase or myeloperoxidase in urine is used as the marker. "Using as a marker for measurement" means "measuring amount or activity". Elastase of a subject means neutrophil elastase and the selective kinetics of neutrophil elastase is used as a marker. The kinetics can use enzyme activity of neutrophil elastase and/or change in amount of neutrophil elastase as a marker. For myeloperoxidase, change in amount thereof can preferably be used as a marker. For analyte, urine can be used. The present examination method is useful for an examination method for monitoring IC, wherein the urine collected from an IC patient is analyzed to identify an azurophilic granular substance in activity or amount (for example, neutrophil elastase in activity and/or amount, or myeloperoxidase in amount), thereby to measure the kinetics (such as presence, increase or decrease) for monitoring. The urine sample used as an analyte in the examination method of the present invention may be intact, diluted or centrifuged. Further, the urine may be frozen and thawed to use. More precisely, the urine is preferably centrifuged to use. Further, it has been known that azurophilic granular substances, in particular neutrophil elastase increases in the urine of a patient with infected urinary disease. In order to exclude such patient, only an urine sample from a patient with uninfected urine disease is preferably selected to use. Therefore, if desired, the examination method of the present invention may comprise a step for determining whether an analyte urine is infected with bacteria. The urine as an analyte, which is infected with bacteria, can reflect the effect of azurophilic granular substances, in particular neutrophil elastase caused by the bacterial infection on the measurement. The bacterial urine can be excluded from the analyte if desired.
Besides a step for identifying anazurophilic granular substance by its activity or amount (in particular, neutrophil elastase by activity and/or amount or myeloperoxidase by amount), a step for analyzing measurements can be comprised in the examination method of the present invention. The step for analyzing measurements means a step for analysis to determine clinical meanings, such as analyzing an obtained results by statistic analysis to define the meaning of the measurements, comparing the measurements of subject's urine sample with those of a healthy person's, comparing the measurements of urine before and after treating the IC patient, analyzing relation with other diseases, and avoiding effects brought by bacterial infection on data.

### Examination method for IC using the amount of an azurophilic granular substance as a marker

The present inventors have found that a high amount of azurophilic granular substances, in particular neutrophil elastase and/or myeloperoxidase is found in urine of an IC patient. Employing this finding, an examination method for IC is established, wherein an azurophilic granular substance (in particular, the amount of neutrophil elastase or/and myeloperoxidase) is measured in urine of a subject.
A quantitative method has already been established for azurophilic granular substances, and they can be determined by a conventional method using their respective specific antibodies (for example, by ELISA method).
For example, to measure neutrophil elastase specifically, immunoassay procedure has already been developed, and such procedure is performed using a specific anti-neutrophil elastase antibody (for example, Human Pathology, 1997, 28 (6): 729-728). Azurophilic granular substances (for example, neutrophil elastase or myoloperoxidase) can be measured by using radioimmunoassay, enzyme immunoassay (for example ELISA) , immunofluorescence, fluorescence-enzyme immunoassay, immunoprecipitation, immuno-agglutination, particle-slide agglutination, dot blow method and the like. In more specifically, they can be measured as follows.
A method for measuring neutrophil elastase by enzyme immunoassay has been known. According to the method, a particular amount of sample is added to an assay container such as a well to which an anti-neutrophil elastase antibody has already been immobilized, and then the assay container is washed to eliminate the other ingredients than neutrophil elastase which is coupled to the immobilized phase. Then, an anti-neutrophil elastase antibody labeled with enzyme (for example, alkaline phosphatase) is mixed and washed again to eliminate the other ingredients than the already coupled neutrophil elastase and the anti-neutrophil elastase antibody further coupled thereto. Finally, the substrate of the label enzyme (for example, 4-nitrophenyl phosphate of alkaline phosphatase substrate) is added to monitor color development as caused by cleavage of the substrate (for example, liberation of 4-nitrophenol) by an absorbance spectrometer. Further, a standard curve can be made with standard solutions comprising a known concentration of elastase to obtain the amount of elastase in the sample. An enzyme immunoassay kit is commercially available (for example, from Immundiagnostik AG) , and can be preferably used in accordance with a method as disclosed in Example 3. The similar approach can also be performed for myeloperoxidase. More specifically, it can be measured in accordance with a method as disclosed in Example 6.
An enzyme immunoassay kit for defensin, which is one of azurophilic granular substances and a bactericidal permeability-increasing protein, is commercially available from Hycult Biotechnology BV, and an enzyme immunoassay kit for cathepsin G is available from American Research Products, Inc. and they can be used.

In order to obtain an antibody to an azurophilic granular substance (for example, neutrophil elastase or myeloperoxidase) to use, various animals (for example, rabbit, rat, goat and chicken) are immunized with an azurophilic granular substance which has been purified by a well known method (for example, neutrophil elastase or myeloperoxidase) or a commercially available purified azurophilic granular substance (for example, neutrophil elastase or myeloperoxidase) to get the polyclonal antibodies. A purified azurophilic granular substance (for example, neutrophil elastase or myeloperoxidase) or the fragment thereof is emulsified in an appropriate adjuvant such as Freund's complete adjuvant to get an emulsion, with which an animal is intraperitoneally, subcutaneously, or intravenously immunized to get serum or egg, thereby to obtain a polyclonal antibody. The polyclonal antibody thus obtained can be separated and purified by a conventional method for isolating and purifying protein. As the conventional method for isolating and purifying protein, for example, centrifugation, dialysis, salt precipitation with ammonium sulfate or chromatography using, for example, DEAE-cellulose, hydroxyapatite or protein A agarose may be mentioned. Further, an anti-azurophilic granular substance (for example, neutrophil elastase or myeloperoxidase) antibody-positive splenocyte produced from an immunized mouse is fused with a mouse myeloma cell by polyethylene glycol to get a hybridoma, from which an anti-azurophilic granular substance (for example, neutrophil elastase or myeloperoxidase) monoclonal antibody is produced to use. The monoclonal antibody and the polyclonal antibody prepared by a well known method can be used and a commercially available product can also be used.

In an immunoactivation (IMAC) method, a homogeneous system of enzyme-labeled Fab' of anti-neutrophil elastase antibody is subjected to an antigen-antibody reaction to give an aggregate, thereby to activate the labeled enzyme, allowing colorimetric determination. The method allows measurement of a complex between free neutrophil elastase and neutrophil elastase-α1PI (α1-protease inhibitor).
In particle-slide agglutination, a latex particle of 3 µm or less, preferably from 0.2 to 1.0 µm is used as a carrier particle. As the method for insolubilizing the anti-azurophilic granular substance (for example, neutrophil elastase or myeloperoxidase) antibody, a common physical adsorption method or a chemical bonding method is used, for example, with water soluble carbodiimide and bifunctional reagent. An F(ab') 2 antibody fragment, which is obtained by removing an Fc part from the anti-azurophilic granular substance (for example, neutrophil elastase or myeloperoxidase) antibody by pepsin treatment, can also be used if needed. A sample containing an azurophilic granular substance (for example, neutrophil elastase or myeloperoxidase) is mixed with a latex reagent sensitized with the anti-azurophilic granular substance (for example, neutrophil elastase or myeloperoxidase) antibody on a slide to cause the antigen-antibody reaction between the azurophilic granular substance (for example, neutrophil elastase or myeloperoxidase) and the anti-azurophilic granular substance (for example, neutrophil elastase or myeloperoxidase) antibody to give aggregates, allowing the visual observation of presence or absence of aggregates, thereby to determine the presence or absence of an azurophilic granular substance (for example, neutrophil elastase or myeloperoxidase) in the sample.

The examination method of the present invention comprises an examination method for IC that includes, besides the step of measuring the amount of an azurophilic granular substance, in particular neutrophil elastase or myeloperoxidase as described above, the step of analyzing measurements thereof. The step of analyzing measurements is preferably a step of comparing the measurements of subject's urine sample with those of a healthy person's. If the measurements of the subject are higher than those of the healthy person, the subject can be determined as a IC patient.
Further, in a more preferable example, the step of analyzing measurements as described above is a step of analyzing whether neutrophil elastase in an urine sample from a subject has a measurement of 3 ng/mL or more (preferably 5 ng/mL or more, and more preferably 10 ng/mL or more) in a condition of Example 3, and 0.2 ng/mL or more (preferably 1 ng/mL or more, and more preferably 5 ng/mL or more) in a condition of Example 5. For example, a subject having a detected neutrophil elastase of 3 ng/mL or more (preferably 5 ng/mL or more, and more preferably 10 ng/mL or more) in the condition of Example 3, and 0.2 ng/mL or more (preferably 1 ng/mL or more, and more preferably 5 ng/mL or more) in the condition of Example 5 can be determined as an IC patient. Further, in a condition of Example 6, a subject having a detected myeloperoxidase of 5 ng/mL or more can be determined as an IC patient.

Further, the examination method of the present invention may be combined with a step of determining whether an urine is infected with bacteria or not, if desired. Namely, the examination method of the present invention includes an examination method for IC which comprises a step of determining whether an urine is infected with bacteria or not, a step of measuring the amount of azurophilic granular substance, in particular neutrophil elastase or myeloperoxidase in the urine sample, and a step of analyzing measurements thereof. Whether the urine is infected with bacteria or not can easily be determined by a well known method. Namely, it can be examined by tests of nitrate (Griess test), glucoseoxidase, catalase (J. Lab. and Clin. Med. 1961 p. 490-494), culture test and the like. More specifically, culture test can be conducted as follows: urine is collected after wiping and cleaning around the ureteric orifice, diluted appropriately as needed, cultured quantitatively, and measured in respect to its urinary bacterial concentration. The urine, which has a bacterial concentration of, for example, 10⁵ colony forming units (CFUs)/mL or more, is examined to give diagnosis of urinary tract infection (Standard Textbook of Laboratory Test Medicine, Jun Igari et al., Igaku-Shoin Ltd.). The step of determining whether the urine is infected with bacteria or not allows discrimination to exclude of an analyte having an increased amount of elastase or myeloperoxidase caused by the bacterial infection. Then, the amount of neutrophil elastase or myeloperoxidase in the urine is measured to analyze the obtained measurements thereof. The step of analyzing measurements is preferably a step of comparing the measurements of subject's urine sample with those of a healthy person's. The urine from a subject, which is not infected with bacteria and has a higher measurement of neutrophil elastase or myeloperoxidase than that from a healthy person, can determine the subject as an IC patient.
Further, in a more preferable example, the step of analyzing measurements described above is a step of analyzing whether neutrophil elastase in the urine sample from a subject has a measurement of 3 ng/mL or more (preferably 5 ng/mL or more, and more preferably 10 ng/mL or more) in the condition of Example 3, or 0.2 ng/mL or more (preferably 1 ng/mL or more, and more preferably 5 ng/mL or more) in the condition of Example 5. For example, the urine from a subject, which is not infected with bacteria and has a measurement of detected neutrophil elastase of 3 ng/mL or more (preferably 5 ng/mL or more, and more preferably 10 ng/mL or more) in the condition of Example 3, or 0.2 ng/mL or more (preferably 1 ng/mL or more, and more preferably 5 mg/mL or more) in the condition of Example 5, can determine he subject as an IC patient. Further, the urine from a subject, which has a measurement of myeloperoxidase of 5 ng/mL or more in the condition of Example 6, can determine the subject as an IC patient.

Examination method for IC using the activity of an azurophilic granular substance as a marker
The present inventors have found that there is an enzyme capable of cleaving a fluorescent peptide substrate [Suc (OMe)-Ala-Ala-Pro-Val MCA] (Ala-Ala-Pro-Val: SEQ ID NO: 1) in the urine of an IC patient. Subsequently, we have identified the enzyme as neutrophil elastase. Employing these findings, we have established an examination method for IC wherein the activity of neutrophil elastase in the urine from a subject is measured. Neutrophil elastase is an azurophilic granular substance, thus we have established an examination method for IC by measuring the activity of azurophilic granular substance.
Methods for measuring the activity of an azurophilic granular substance have already been established and, for example, the method for measuring the activity of cathepsin G, which is one of azurophilic granular substance, is reported in Biol. Chem., 1992, 267: 21712-21719, for myeloperoxidase in Arzneimittelforschung., 2004, 54 (7): 389-395, for protease 3 in J. Immunol., 2000, 165: 3366-3374, for Defensin in J Clin. Invest. 1998, 101: 178-187 and for a Bactericidal permeability-increasing protein in J. Leukoc., Biol., 1998, 64: 14-18.
A method for measuring the activity of neutrophil elastase has also been known (Biochemistry Experiments 30, Protease I, p. 27-33). The activity of neutrophil elastase can be measured by measuring the degradation of 3H-elastin or Congo red-elastin, or by measuring the degradation of the specific synthetic substrate. While elastin, collagen, proteoglycan, fibronectin and the like are known to be degraded by neutrophil elastase, the preferable substrate for use in the method of the present invention is a substrate specific for neutrophil elastase, and particularly preferably is a synthetic substrate because of its easiness of operation. As the substrate for neutrophil elastase, many specific peptide derivatives are widely known, and, for example, Suc (OMe)-Ala-Ala-Pro-Val-pNA, Suc (OMe)-Ala-Ala-Pro-Met-pNA (Ala-Ala-Pro-Met : SEQ ID NO:2), Suc (OMe)-Ala-Ile-Pro-Met-pNA (Ala-Ile-Pro-Met: SEQ ID NO: 3) and pyroGlu-Pro-Val-pNA are mentioned. Among them, the most preferred substrate is the fluorescent peptide substrate (Suc (OMe)-Ala-Ala-Pro-Val MCA), which was used in Examples 1 and 2. These peptide substrates can be made according to, for example, Chemical Synthesis Method of Peptide (protein IV (New Biochemistry experiments, Course 1), The Japanese Biochemical Society Ed., p. 3-74, Tokyo Kagaku Dozin Co., Ltd. (1992)) which uses a liquid phase or solid phase method. Hydrolysis of a substrate can be continuously monitored by regularly observing fluorescence generated by degradation. Therefore, measurement and analysis can be conducted after a defined time or at the end point of the reaction when the substrate is completely hydrolyzed.

The present invention comprises an examination method for IC characterized by including a step of measuring the activity of an azurophilic granular substance (for example, neutrophil elastase) in the urine sample from a patient as a subject with uninfected urine disease, and a step of analyzing the measurements thereof. The step of analyzing measurements is preferably a step of comparing the measurements of subject's urine sample with those of a healthy person's. The urine from a subject, which has a higher measurement than that from a healthy person, can determine the subject as an IC patient. Further, in a preferable example, for example, the urine from a subject, which has a measurement of neutrophil elastase activity of 0. 5 ng/mL standard enzyme activity (more preferably 1 ng/mL standard enzyme activity) or more in the condition of Example 4, can determine the subject as an IC patient.

Further, the examination method of the present invention includes an examination method for IC, comprising the step of determining whether an urine is infected with bacteria or not, the step of measuring the activity of neutrophil elastase in the urine sample, and the step of analyzing measurements thereof. Whether the urine is infected with bacteria or not can easily be determined by a well known method described above. The urine from a subject, which is not infected with bacteria and has a higher measurement of an azurophilic granular substance (for example, neutrophil elastase) activity than that from a healthy person, can determine the subject as an IC patient. Further, in a preferable example, the urine from a subject, which has a measurement of neutrophil elastase activity of 0. 5 ng/mL standard enzyme activity (more preferably 1 ng/mL standard enzyme activity) or more in the condition of Example 4, can determine the subject as an IC patient.

### Examination kit for IC

The present invention includes an examination kit for IC for use in the above described examination method for IC, which uses the activity of neutrophil elastase as a marker, comprising an assay container, a buffer and a neutrophil elastase substrate, and also an examination kit for IC for use in the above described examination method for IC, which uses the amount of an azurophilic granular substance, in particular neutrophil elastase or myeloperoxidase as a marker, comprising an assay container and an anti-azurophilic granular substance antibody , in particular an anti-neutrophil elastase antibody or an anti-myeloperoxidase antibody.
An appropriate kit for performing the examination method for IC of the present invention by measuring the activity of neutrophil elastase comprises, for example, an assay container, a buffer and a neutrophil elastase substrate. The kit having a preferable form comprises a plate having a plurality of wells as an assay container. The plate is preferably a plate which can easily detect changes after assay, and many assay plates (for example, 96-well plate) are widely known. As the buffer, for example, Tris hydrochloric acid or sodium phosphate solution which have a buffering capacity for a neutral or basic region can be used, and a buffer described in Example 1 can be preferably mentioned.
An appropriate kit for performing an examination method for IC of the present invention by measuring the amount of an azurophilic granular substance, in particular neutrophil elastase or myeloperoxidase, comprises an assay container and an anti-azurophilic granular substance antibody, in particular an anti-neutrophil elastase antibody or an anti-myeloperoxidase antibody (preferably, an antibody able to specifically recognize neutrophil elastase or myeloperoxidase). The kit having a preferable form comprises a plate having a plurality of wells. The plate is preferably a plate which can easily detect changes after assay, and many assay plates (for example, 96-well plate) are widely known. Further, a wash solution and neutrophil elastase or myeloperoxidase to be used as a standard protein can be contained in the kit. By using a standard protein, the amount of neutrophil elastase or myeloperoxidase can be measured.

### The use of an azurophilic granular substance as an IC test marker or an evaluation marker for pharmacological effects

The novel use of an azurophilic granular substance as an IC test marker or an evaluation marker for pharmacological effects is also an object of the present invention. As indicated above, an azurophilic granular substance, in particular neutrophil elastase or myeloperoxidase shows a significant dynamic kinetic in an IC patient, and thus can be used as a test marker to determine IC or as a marker to evaluate pharmacological effects of a drug in an appropriate treatment made on an IC patient or a screening test made on an animal. For example, in order to compare a subject and a healthy person, the measurement of an azurophilic granular substance, in particular neutrophil elastase or myeloperoxidase in urine is used as the marker. The urine from the subject, which has a higher measurement than that from the healthy person, can determine the subject as an IC patient. Namely, azurophilic granular substances, in particular neutrophil elastase or myeloperoxidase can be used as the marker in determination for examination of IC. Further, in order to allow evaluation of the pharmacological effects of a candidate drug, for example, an IC patient or animal model with IC disease is administered with the drug, and an azurophilic granular substance, in particular neutrophil elastase or myeloperoxidase is used as an evaluation marker to compare the measurements thereof before and after the administration. Namely, an azurophilic granular substances, in particular neutrophil elastase or myeloperoxidase can be used as the marker for evaluating pharmacological effects of a drug administered for IC disease. In order to allow examination of IC, the urine from a subject is analyzed to determine whether neutrophil elastase has a measurement of 3 ng/mL or more (preferably 5 ng/mL or more, and more preferably 10 ng/mL or more) in the condition of Example 3 and 0. 2 ng/mL or more (preferably 1 ng/mL or more, and more preferably 5 ng/mL or more) in the condition of Example 5 or not, or whether myeloperoxidase has a measurement of 5 ng/mL or more in the condition of Example 6 or not. For example, the subject, who is detected to have an urinary neutrophil elastase of 3 ng/mL or more (preferably 5 ng/mL or more, and more preferably 10 ng/mL or more) in the condition of Example 3, and 0.2 ng/mL or more (preferably 1 ng/mL or more, and more preferably 5 ng/mL or more) in the condition of Example 5, can be determined as an IC patient. Further, the subject, who is detected to have an urinary myeloperoxidase of 5 ng/mL or more in the condition of Example 6, can be determined as an IC patient. The urinary measurement from a patient identified as an IC patient is compared with that from the patient administrated with a drug after a certain period, allowing evaluation of the drug in pharmacological effects. Therefore, an azurophilic granular substance can be used as the evaluation marker for pharmacological effects.

### The examination method for therapeutic effects on IC patient using an azurophilic granular substance as a marker

Further, the present invention also is an examination method for evaluating the effects of a therapy on an IC patient using an azurophilic granular substance as a marker. As indicated above, azurophilic granular substances, in particular neutrophil elastase or myeloperoxidase shows a significant dynamic kinetic in an IC patient, thus it is meaningful to use an azurophilic granular substance as a marker for evaluating the effects of a therapy conducted appropriately on an IC patient. As observed in Example 7, a correlation is confirmed between clinical symptoms and examination values of an azurophilic granular substance, and a high elastase concentration was especially found in a group suffering from pain. Namely, the correlation was confirmed between a high pain score among symptom scores in an IC patient group and a high elastase concentration. From this fact, it is proved that the concentration of an azurophilic granular substance, in particular elastase is measured to serve as a marker for improving symptoms.
Therapy includes, besides dosing, any therapeutic means accessible for IC treatment. An IC patient or an animal model with IC disease is applied with a specific therapy to compare the measurements of an azurophilic granular substance, in particular neutrophil elastase or myeloperoxidase before and after the application, allowing evaluation of the effects of the therapy.
Namely, the present application includes an examination method for therapeutic effects on a patient with interstitial cystitis, comprising a step of measuring the amount or the activity of an azurophilic granular substance (preferably, neutrophil elastase or myeloperoxidase) as the marker. Further, the present application includes an examination method for therapeutic effects on a patient with interstitial cystitis, comprising a step for using the urines of an patient as analytes before and after the therapy of IC to measure the amount or the activity of an azurophilic granular substance (preferably, neutrophil elastase or myeloperoxidase) as a marker, and a step of comparing both measurements.
Judging from the result of Example 7, an example is mentioned as follows: firstly a subject, who has a detected urinary neutrophil elastase of 1 ng/mL or more (preferably 5 ng/mL or more, and more preferably 10 ng/mL or more), can be determined as an IC patient, and then the urinary neutrophil elastase measurements of the patient are compared between before and after a certain period of therapy to determine whether a decrease or a change can be seen between them or not, allowing evaluation of the effect of the therapy. Namely, an azurophilic granular substance is measured as a marker to provide an effective examination method for therapeutic effects on an IC patient.

### (Example)

The present invention will be explained with Examples below, but is not limited to them. Meanwhile, unless otherwise mentioned, Examples can be performed according to a well known method (for example, Methods in Enzymology, Academic Press, Inc.). Further, if a commercially available reagent and kit are used, Examples can be performed according to the instruction for a commercially available product.

### Example 1

### Detection of proteolytic enzyme activity present in urine of interstitial cystitis (IC) patient

5.3 mg of the fluorescent peptide substrate [Suc (OMe)-Ala-Ala-Pro-Val MCA; Peptide Institute, Inc.] was dissolved in 850 µl of dimethyl sulfoxide to get a Solution, which was then used as a stock solution of the fluorescent peptide substrate. A buffer prepared to have a final concentration of 0.2 M triethanolamine buffer (pH 8.0)/ 1 M sodium chloride/0.02% sodium azide /0.1% polyethylene glycol 6000 was added into a 96-well plate (EIA/RIA plate, Cat. No. 3694; Costar), and then the stock solution of fluorescent peptide substrate and urine from an IC patient or a healthy person were added to have their respective final concentrations of 0.5% and 25%, to conduct an enzyme reaction. In this example, collected urine was not centrifuged, but cryopreserved and then thawed to use as a measurement sample. The solution of 100 µl was used for the reaction. Further, the reaction was conducted in an incubator at 37°C. After overnight enzyme reaction, fluorescence was measured using a fluorescence platereader (SAFIRE; Tecan Group Ltd.). Measurement was conducted by measuring 460 nm fluorescence released from 380 nm exciting light, and the increase of fluorescence was observed to detect a proteolytic enzyme activity.
As a result, proteolytic enzyme activity was detected in 7 of 17 urines from IC patients (their measurements≥5000) , but in none but one of 11 urines from healthy subjects (10 urines having measurements≤1500). It was found by these facts that proteolytic enzyme activity in urine can be measured to examine an IC patient.

### Example 2

### Purification and identification of urinary protease

Activity for degrading the fluorescent peptide substrate used in Example 1 was used as an indicator to purify the protease from the urine of an IC patient. 100 ml of cryopreserved urine from the IC patient was thawed, and centrifuged at 500 x g for 5 minutes to obtain a supernatant, which was then further filtered through a 0.22 µm filter paper to eliminate insoluble matters. 100 ml of 20 mM Tris-HCl/0.5 M NaCl (pH 7.4) was added thereto to prepare a ConA Sepharose loading fraction. A column was previously filled with 1 ml of ConA Sepharose (Amersham Bioscience), which was equilibrated with 20 mM Tris-HCl/0.5 M NaCl (pH 7.4) and loaded with 200ml of above described loading fraction at a flow rate of approximately 0.5 ml per minute. At this time, the solution running through the column was defined as a ConA Sepharose unadsorbed fraction. After the loading fraction was completely passed through the column, contaminants which did not bind to ConA Sepharose were washed away with 10 ml of 20 mM Tris-HCl/0.5 M NaCl (pH 7.4). Then, the protein which bound to ConA Sepharose was eluted with 10 ml of 20 mM Tris-HCl/0.5 M NaCl/0.5 M α-D-methylmannoside (pH 7.4) to get a ConA Sepharose eluted fraction. The ConA Sepharose loading fraction, the ConA Sepharose unadsorbed fraction, and the ConA Sepharose eluted fraction were analyzed instead of urine in the way as in Example 1 to measure their respective proteolytic enzyme activities. As a result, it was confirmed that the protease present in the ConA Sepharose loading fraction was adsorbed onto and eluted from the ConA Sepharose to recover in the ConA Sepharose eluted fraction.
The ConA Sepharose eluted fraction was centrifuged at 5000 x g for 10 minutes using Amicon Ultra-15 MWCO 10K (Millipore) to concentrate into 1ml of solution. The solution was supplied with 9 ml of 50 mM Tris-HCl /0. 1% CHAPS (pH 8.0), and centrifuged again to concentrate into 1ml of solution. The solution was supplied with 9 ml of 50 mM Tris-HCl/0.1% CHAPS (pH 8.0) again, and centrifuged to concentrate into 1 ml of solution, which was defined as a MonoQ loading fraction. The following purification operation was conducted using AKTAexplore (Amersham Bioscience). Mono Q HR5/5 (Amersham Bioscience) was equilibrated with 50 mM Tris-HCl and 0.1% CHAPS (pH 8.0) in advance, and loaded with the MonoQ loading fraction at a flowrate of 0. 5 ml per minute. At this time, the fraction which did not adsorb to the column was defined as a MonoQ unadsorbed fraction. Then, contaminants in the column was washed away with a 10 ml of 50 mM Tris-HCl /0.1% CHAPS (pH 8.0) buffer. In order to elute, 50 mM Tris-HCl /0.1% CHAPS (pH 8.0) was time-dependently converted to 50 mM Tris-HCl/0.1% CHAPS/1 M NaCl (pH 8.0) to make a linear concentration gradient of NaCl. For the concentration gradient, the NaCl concentration was programmed to change from 0 M to 1 M for 50 minutes. Elution was conducted at a flow rate of 0.5 ml per minute, and 0.5 ml of eluted fraction was collected for 1 minute, thereby to obtain totally 50 fractions. The MonoQ loading fraction, the MonoQ unadsorbed fraction and the MonoQ eluted fraction were analyzed in the way as in Example 1 to measure their respective proteolytic enzyme activities. As a result, it was confirmed that the observed activity of the loading fraction was adsorbed onto the MonoQ column and eluted with 0.2 M to 0.6 M NaCl. Approximately 5 ml of the fractions, which had been eluted with about 0. 3 M to 0.5 M NaCl to have especially strong activities among the eluted fractions with confirmed activities, were concentrated into 200 µl using AmiconUltra-4 MWCO 10K (Millipore).
Superdex200HR10/30 (Amersham Bioscience) previously equilibrated with 50 mM Tris-HCl/0.1% CHAPS/1 M NaCl (pH 8.0) was loaded with the concentrated fraction to conduct molecular weight fractionation. The loaded sample was eluted with 50 mM Tris-HCl/0.1% CHAPS/1 M NaCl (pH 8.0) flowing at 0. 5 ml per minute to collect every 0. 5 ml of eluted solution from the column for 60 minutes. Each of the eluted fractions was analyzed in the way according to Example 1 to measure their respective proteolytic activities. As a result, the fractions eluted at any time of 38 to 48 minutes were found to have strong activities. The fraction confirmed to have a strong activity was concentrated using AmiconUltra-4 MWCO 10K (Millipore) into 100 µl of solution. 15 µl of the solution was subjected to SDS polyacrylamide gel electrophoresis (SDS-PAGE) under a reductive condition. The SDS-PAGE was conducted in accordance with the method by Laemmli et al. (Nature 1970, 227: 680-685) . Migration was conducted at 40mA of constant current for 60 minutes using PAG mini [Daiichi] 4/20 (Daiichi Pure Chemicals Co. , Ltd.) as a gel. The gel after running was silver stained with Silver Stain MS Kit (Wako Pure Chemical Industries, Ltd.). Staining was conducted according to the attached instruction and using the attached reagents.
Protein stained on the gel was excised from the gel by a scalpel. The protein contained in an excised gel piece was fragmented in the gel by the enzyme digestion method (Schevchenko et al., Analytical Chemistry, 1996, 68: 850-858) using trypsin to collect a peptide mixture from the gel.
The obtained peptide mixture was separated into each peptide by the acetonitrile gradient elution method using a capillary reverse phase liquid chromatography column (PepmapC18, particle size of 3 µm, inside diameter of 0.075 mm, length of 150 mm, from LC Packings), in the presence of 0.1% formic acid, at a flow rate of approximately 200 nl per minute. The molecular ions of each peptide were automatically selected by the quadrupole-time-of-flight hybrid mass spectrometry apparatus (Q-Tof UltimaAPI, Micromass) having an electrospray ion source directly interfaced to the liquid chromatograph apparatus (CapLCTM, Micromass) , and then the product ion spectra of each peptide were obtained by a method for measuring their product ion spectra (Survey scan method).
The product ion spectra of each peptide in the peptide mixture obtained by trypsin digestion in the gel described above were searched to verify using a public protein database Refseq (release 20030830, human sequence) by analysis software Mascot (Matrix Science KK.). As a result, there were found the peptides which coincide to two partial amino acid sequences [VVLGAHNLSR (SEQ ID NO: 4) and ARPHAWPFMVSLQLR (SEQ ID NO: 5)] in neutrophil elastase (Elastase 2, neutrophil, Refseq accession No. NP_001963), thus the protein in the gel was revealed to be neutrophil elastase. It is demonstrated by these facts that proteolytic enzyme activity in the urine of an IC patient is based on neutrophil elastase, and that the urine is analyzed to measure proteolytic enzyme activity, in particular, the activity of neutrophil elastase, allowing examination of an IC patient.

### Example 3

### Measurement of neutrophil elastase in urine by enzyme immunoassay

Based on the facts that the fluorescent peptide substrate used in Example 1 was degradable by neutrophil elastase, and that neutrophil elastase was purified from the urine of an IC patient and identified using the activity for degrading the fluorescent peptide substrate described above as an indicator, the urines from an IC patient and a healthy person were used to measure their contents of neutrophil elastase. The neutrophil elastase enzyme immunoassay kit (Immundiagnostik AG) was used to measure with the attached reagents according to the attached instruction. In this example, the collected urine was not been centrifuged, but cryopreserved and thawed for use. The urines from the IC patient and the healthy person were diluted tenfold with the attached wash solution to prepare the samples for measurement. Further, the attached standard protein (neutrophil elastase) and the wash solution were used to prepare standard protein solutions of 0, 0.1, 0.3, 1, 3 and 10 ng/ml standard protein. To the attached 96-well plate to which the anti-neutrophil elastase antibody had already been immobilized, 100 µl of standard protein solutions or the samples for measurement were added, and shaken at room temperature for an hour to facilitate the antibody binding reaction. The plate was washed five times with 250 µl of wash solution to wash unbound protein away, then supplied with 100 µl of a solution of anti-neutrophil elastase antibody (mouse-derived monoclonal) which had been previously diluted according to the attached instruction, and shaken at room temperature for an hour to facilitate the antibody binding reaction. The plate was washed five times with 250 µl of wash solution to wash unbound antibody away, then supplied with 100 µl of solution of peroxidase-labeled antimouse immunoglobulin antibody which had been previously diluted according to the attached instruction, and then shaken at room temperature for 30 minutes to facilitate the antibody binding reaction. The plate was washed five times with 250 µl of wash solution to wash unbound antibody away, then supplied with 100 µl of the attached tetramethylbenzidine (TMB) substrate, and then shaken at room temperature for 15 minutes to facilitate the enzyme reaction. After 50 µl of the attached reaction stop solution was added, the absorbance was measured at 450 nm using a platereader (SpectraMAX250; MolecularDevice). A standard curve was made based on the measurements of the standard proteins, and neutrophil elastase content in the sample for measurement was calculated based on the standard curve.
As a result, 13 of 17 urines from IC patients were detected to have a urinary neutrophil elastase amount of 3 ng/ml or more respectively, while none of 11 from healthy persons were detected to have a urinary neutrophil elastase amount of 3 ng/ml or more respectively. Further, 7 of 17 urines from IC patients were detected to have a urinary neutrophil elastase amount of 10 ng/ml or more respectively, and completely coincided with the urines from the IC patients which were detected to have proteolytic enzyme activity. It was found from these facts that the amount of neutrophil elastase in urine can be measured to examine the IC patient.

### Example 4

### More accurate detection of proteolytic enzyme activity present in urine of IC patient

Urines collected from IC patients and healthy persons sometimes contain blood cells such as neutrophil. These cells are broken by freeze and release intracellular proteins into the urines. If the urine contains neutrophils, the cells may likely to release neutrophil elastase into the urine, thereby to affect urinary activity to measure. Therefore, collected urine was centrifuged by a centrifuge machine to give a supernatant after precipitation of the cells, which was then used to measure the proteolytic enzyme activity.
Further, urine contains a substance which is irradiated with 380 nm exciting light to emit 460 nm fluorescence. Thus, proteolytic enzyme activity was measured by a following method for correcting a background. There were previously prepared a substrate-containing buffer and a substrate-free buffer, each of which was supplied with an urine and left to stand for 2 hours to measure the fluorescence value. The substrate-containing measurement solution gave one fluorescence value which a degraded substrate and the urine were united to have. On the other hand, the substrate-free measurement solution gave another fluorescence value which only the urine had. Therefore, the latter fluorescence value was subtracted from the former fluorescence value to give a difference, that is, a fluorescence value which only the degraded substrate had. Thus, the value could be calculated to give a urinary proteolytic enzyme activity more accurately.
This example used the method, the reagents, the apparatus and the like as used in Example 1, except that the reaction time was set to be 2 hours. Human neutrophil elastase (Elastin Products Company, Cat. No. CK828) was used to measure as a standard enzyme. Simultaneously, 0, 0.3125, 0. 625, 1.25, 2.5, 5, 10 and 20 ng/ml of the standard enzyme solutions were used to determine the corresponding measurements, thereby to make a standard curve. A urinary proteolytic enzyme activity to measure was calculated based on the standard curve.
As a result, concerning newly measured samples, 47 of 67 urines from IC patients were detected to have a standard enzyme activity of 0.5 ng/ml or more respectively. On the other hand, of 9 urines of healthy persons, only 1 case was detected to have a standard enzyme activity of 0.5 ng/ml or more, while 8 cases were below the detection limit respectively.
It was found that, in a method for measuring a urinary elastase activity, there could be used an urine sample which an urine had not been centrifuged but freeze-thawed to give as done in Example 1, as well as a supernatant sample which a collected urine (stock urine) had been centrifuged to give as done in this example. Further, it was found that a background could be corrected to measure a urinary elastase activity more accurately.

### Example 5

### More accurate measurement of neutrophil elastase in urine by enzyme immunoassay

For measurement, an urine was previously centrifuged to eliminate cells in the way as in Example 4. This example used the method, the reagents, the apparatus and the like as used in Example 3, except that the urine was diluted twofold to measure as a sample. As a result, concerning newly measured samples, 43 of 67 urines from IC patients were detected to have a neutrophil elastase of 0.2 ng/ml or more respectively. Further, concerning urines of healthy persons, of 9 urines of healthy persons, only 1 urine was detected to have a neutrophil elastase of 0.2 ng/ml or more, while 8 urines were below the detection limit respectively. It was found that, in a method for measuring the urinary elastase amount, an urine sample which a urine had not been centrifuged but freeze-thawed to give as done in Example 3, as well as a supernatant sample which a collected urine (original urine) had been centrifuged to give as done in this example could be used to measure a urinary elastase amount more accurately.

### Example 6

Measurment of myeloperoxidase by enzyme immunoassay The amount of myeloperoxidase present in urine was measured by enzyme immunoassay. The measurement was conducted with myeloperoxidase enzyme immunoassay kit (Immundiagnostik AG) according to the attached instruction using the attached reagents. The urines from the IC patient and the healthy person were diluted tenfold with the attached wash solution to prepare samples for measurement. Further, the attached standard protein (myeloperoxidase) and the wash solution were used to prepare standard protein solutions of 0, 3.6, 11, 33 and 100 ng/ml standard protein. The attached 96-well plate to which the anti-myeloperoxidase antibody had already been immobilized was washed five times with 250 µl of wash solution. 100 µl of standard protein solutions or the samples for measurement were added to the plate, and shaken at room temperature for an hour to facilitate the antibody binding reaction. The plate was washed five times with 250 µl of wash solution to wash unbound protein away, then supplied with 100 µl of a solution of peroxidase-labeled anti-myeloperoxidase antibody (polyclonal antibody) which had been previously diluted according to the attached instruction, and shaken at room temperature for an hour to facilitate the antibody binding reaction. The plate was washed five times with 250 µl of wash solution to wash unbound antibody away, then supplied with 100 µl of the attached tetramethylbenzidine (TMB) substrate, and then shaken at room temperature for 15 minutes to facilitate the enzyme reaction. After 50 µl of the attached reaction stop solution was added, the absorbance was measured at 450 nm using a plate reader (SpectraMAX250; MolecularDevice). A standard curve was made based on the measurements of the standard proteins, and myeloperoxidase content in the sample for measurement was calculated based on the standard curve.
As a result, 8 of 17 urines from IC patients were detected to have a urinary myeloperoxidase amount of 5 ng/ml or more respectively, while none but one from 11 healthy persons were detected to have a urinary myeloperoxidase amount of 5 ng/ml or more respectively. It was found from these facts that the amount of myeloperoxidase in urine could be measured to examine the IC patient.
The urines from IC patients used as sample in this example were the same as used in Example 1. Of 8 urines from IC patients which were detected to have a myeloperoxidase amount of 5 ng/ml or more respectively, 6 urines coincided with the samples which were determined to have proteolytic enzyme activity (measurement≥5000) (Example 1). Further, all of 8 urines from IC patients which were detected to have a myeloperoxidase amount of 5 ng/ml or more respectively coincided with the samples which were detected to have a neutrophil elastase amount of 3 ng/ml or more as measured by enzyme immunoassay respectively (Example 3). Thus, the amount of myeloperoxidase present in urine was correlated with the amount of neutrophil elastase in urine. Meanwhile, it is known that neutrophil elastase and myeloperoxidase were included in azurophilic granules. It was found by these fasts that the amount of azurophilic granular substance present in urine could be measured to determine an IC patient.

### Example 7

### Use as an evaluation marker for pharmaccological effects or evaluation marker for therapeutic effects

At present, in order to evaluate therapeutic effect clinically, a patient's subjective symptom is used as an end point to improve. However, an objective marker, which could evaluate quantitatively therapeutic effect, would be quite useful for judging the effect. In order to judge symptoms of IC, the symptom score (Symptom Index) (Urology 1997, 49: 58-63) is used in a table of questionnaire. In this score, an IC symptom is judged by urgency, frequent urination, nocturia, and bladder pain, each of which is further divided into 5 stages. Urinary neutrophil elastase level, which could be acknowledged to correlate with these scores, would be measured to serve as a useful marker for symptom improvement. Thus, 67 IC patients in Example 4 (Table 1) and Example 5 (Table 2) were analyzed to classify into a pain group and a pain-free group. As a result, it was revealed that the pain group had a higher activity or amount of neutrophil elastase. Namely, the activity or amount of neutrophil elastase was found to highly correlate with the pain score among symptom scores, suggesting that the activity or amount of neutrophil elastase can become a marker for symptom improvement.
Meanwhile, data in Tables 1 and 2 were calculated based on the results of 60 IC patients with pain and 7 IC patients without pain.

**Table 1**

| **ng/mL activity** | **IC (pain-free group)** | **IC (pain group)** |
|---|---|---|
| **ND** | **57.1%** | **26.7%** |
| **~1** | **28.6%** | **11.7%** |
| **1~10** | **14.3%** | **50.0%** |
| **10~100** | **0.0%** | **8.3%** |
| **100~** | **0.0%** | **3.3%** |

**Table 2**

| **ng/mL** | **IC (pain-free group)** | **IC (pain group)** |
|---|---|---|
| **ND** | **85.7%** | **30.0%** |
| **~1** | **14.3%** | **25.0%** |
| **1~10** | **0.0%** | **35.0%** |
| **10~100** | **0.0%** | **8.3%** |
| **100~** | **0.0%** | **1.7%** |

## Claims

1. A method for examining interstitial cystitis comprising a step for measuring azurophilic granular substance as a marker.

2. The examination method according to Claim 1, wherein the azurophilic granular substance is neutrophil elastase.

3. The examination method according to Claim 1, wherein the azurophilic granular substance is myeloperoxidase.

4. The examination method according to any one of Claims 1 to 3, wherein the urine sample from a patient with an uninfected urine disease as a subject is used as an analyte for the examination.

5. The examination method according to any one of Claims 1 to 4, comprising further a step of determining whether the urine sample is infected with a bacteria or not.

6. The examination method according to any one of claims 2, 4 and 5, wherein the step of measuring neutrophil elastase as a marker is a step for measuring neutrophil elastase amount.

7. The examination method according to any one of Claims 2, 4 and 5, wherein the step of measuring neutrophil elastase as a marker is a step for measuring neutrophil elastase activity.

8. The examination method according to Claim 6, wherein the step for measuring neutrophil elastase amount is a step conducted by immunoassay.

9. The examination method according to claim 7, wherein the step for measuring neutrophil elastase activity is a step conducted with a synthetic substrate.

10. A kit for examining interstitial cystitis for use in the examination method according to Claim 8, comprising an assay container and an anti-neutrophil elastase antibody for measuring neutrophil elastase amount.

11. A kit for examining interstitial cystitis for use in the examination method according to Claim 9, comprising an assay container, a buffer and a neutrophil elastase substrate for measuring neutrophil elastase activity.

12. A use of an azurophilic granular substance as a marker for examining interstitial cystitis or for evaluating pharmacological effects of a drug.

13. A method for examining therapeutic effects on a patient with interstitial cystitis using an azurophilic granular substance as a marker.
